# EUROPEAN PATENT APPLICATION

(11) **EP 0 885 603 A2**
(43) Date of publication of application: **23.12.1998**
(21) Application number: 98203080.1
(22) Date of filing: 17.09.1993
(51) Int. Cl.: A61F 13/02

(54) **A method of manufacturing plasters**

(62) Divisional of application: 93307348.8
(71) Applicant: Tanaka, Nobutaka, Daito-shi, Osaka-fu (JP)
(72) Inventor: Tanaka, Nobutaka, Daito-shi, Osaka-fu (JP)
(74) Representative: Smith, Norman Ian

(57) **Abstract**

A plaster which is applied to the trouble part on the human body in order to abate a muscular ache or swelling. The plaster is made of a mesh-like sheet (1) having an adhesive face and perforated with many uniform square or rectangle or lozenge holes (10). One side of the hole measures in the range of from 3 millimeters to 7 millimeters and an interval between the facing sides of two holes measures in the range of from 2.5 millimeters to 3 millimeters.

## Description

This invention relates to a method of manufacturing plasters which are applied to a trouble part of the human body in order to relieve muscular ache or swelling.

A taping therapy, which means that plasters are applied to trouble parts on the human body, is well known as one of the method of medical treatment for a sprain or a bruise. This medical treatment is operated at an oriental osteopathy clinic where an oriental medical therapy such as an acupuncture, a cauterisation with moxa, and a finger-pressure treatment are in effect.

A curative value by the taping treatment can be proved by means of a gate control theory, an axis cylinder reflex theory, a somatic autonomic reflex theory, and the others. Concretely they say that a fascia protective action by means of the taping therapy enhances the curative value.

Generally, a strain of muscle is often caused by a lack of exercise, an external shock, senility, stress and so on. And then a muscular ache is caused by the strain of muscles. In order to endure the pain, the strain of the muscles is invited again, and the condition of the disease develops into a myalgia by the muscular rigor and developes further into a cellulitis .

An usual plaster used for the taping treatment in order to abate the muscular ache or swelling is a simple non-pored sheet having an adhesive face.

Otherwise,the Oriental medical science says that there are a large number of important points on the human body ,which are connected with the internal organs or tissues and control their functions . These points are called "tsubo" in Japan.

The non-pored sheet is applied to the disease parts or the "tsubo" related to the illness on the human body. Thereby the pain of the muscles can be relieved .

Above mentioned usual plaster,however,is not sufficient for the remedial value.

The problem is as follows;

The former non-pored plaster have a function to protect the malady parts or the "tsubo" related to the disease on the human body. Thereby,a burden on the trouble parts or the "tsubo" can be relieved,and then a healing phenomenon by a natural healing force can proceed smoothly in the human body .

However,since the non-pored plaster is no more than a protective sheet which protect the disease parts on the human body until natural recovery from disease , it is difficult to expect a positive and early recovery. Accordingly the remedial value by the former non-pored plaster is still inadequate.

In view of the foregoing,an object of the invention is to provide a plaster which is applied to a disease part involving a pain or a swelling or to a "tsubo" related to the illness on the human body and which can hasten the recovery of the ailment.

Examples of plasters are disclosed in the following patents/applications: US 3,214,502; US 2,096,564; US 2,807,262; CH 332,999; DE 2,517,790. In these disclosures the perforations are provided to encourage ventilation, to reduce irritation and/or to allow movement. Some of these documents also disclose methods of manufacturing plasters with peforations.

In a method disclosed in US 3,214,502 perforations are made by applying heat and pressure to an adhesive layer and a backing layer. The material of the two layers melt to form perforations through the plaster.

CH 332,999 discloses a method in which small perforations are made using needle shaped spikes mounted on raised portions which are used to remove the adhesive in the region of the perforations.

DE 2,517,790 discloses a method in which series of slits are cut in an adhesive strip and then the strip is heat treated to widen out the slits to give the desired openings.

According to the present invention there is provided a method of manufacturing plasters for application to a troubled or damaged part of the human body in order to abate a muscular ache or swelling;
said plasters being made of a mesh-like sheet having an adhesive face and being perforated with a plurality of generally uniform holes;
   said method comprising the step of,
cutting a plurality of slits in sheet; and being characterised by the further steps of,
supplying said sheet which comprises main sheet having an adhesive face and separate paper covering the adhesive face; and
using a punching roll in said cutting step to cut said plurality of slits in said main sheet without cutting said separate paper, said plurality of slits corresponding to outlines of said generally uniform holes.

The function of the plaster will be clarified in detail. The plaster made of the mesh-like sheet is applied to the disease part on the human body. Thus, since the disease part is covered by the mesh-like plaster, the disease part on the human body is protected by it as same as by the above mentioned usual non-pored plaster. However, when the applicated condition of the plaster in this invention to the human body is observed in detail, there is a distinction between the mesh-like sheet and the non-pored sheet. An adhesion of the mesh-like sheet is concentrated under lines around meshes and under the cross-points of the lines. Namely, the adhesion of the plaster acts to the trouble part on the human body partially, in other words, the adhesion of the mesh-like plaster operates to the disease part on the human body as a dynamical burden.

The trouble is caused by the very strong strain or relaxation of the muscles, and when the mesh-like plaster is applied to the trouble part on the human body, the muscular strain by the adhesive parts of the plaster and the muscular relaxation by the un-adhesive parts of the plaster produce good strained condition to the muscles. Namely, a formation of a sodium element in the disease part can be promoted by the dynamical burden and a satiration period of the sodium element is advanced. Accordingly a neutralization starting from the satiration period of the sodium element i.e. the natural healing fenomenon can start and advance in an early stage, and the pain of the muscles abates in the early time.

A consequence of a comparison test which compares the remedial value by the mesh-like plaster in this invention with by the former non-pored plaster will explain as shown in a table 1.

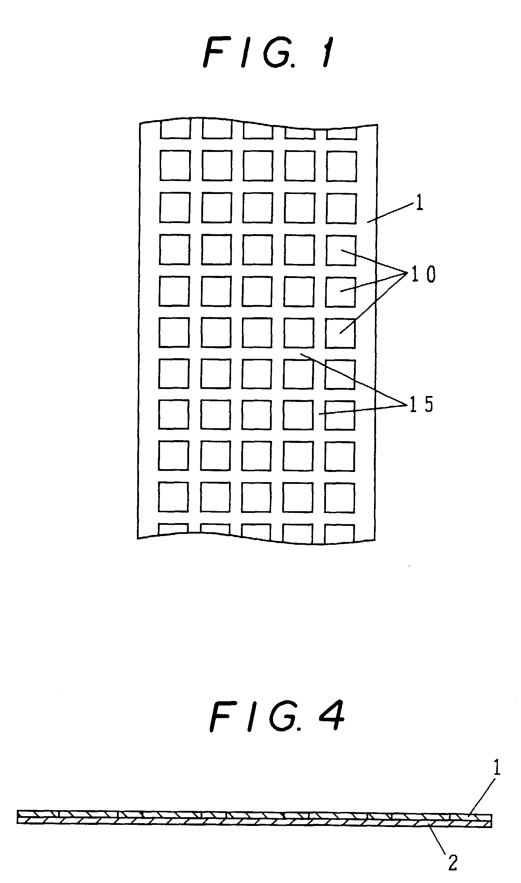

A plaster used in the comparison test is perforated with many square holes uniformly as shown in Fig.1. The plaster is 57 millimeters in width and one side of the square hole is 7 millimeters long. And the square holes are arranged at 3-millimeter intervals lengthwise and sidewise.

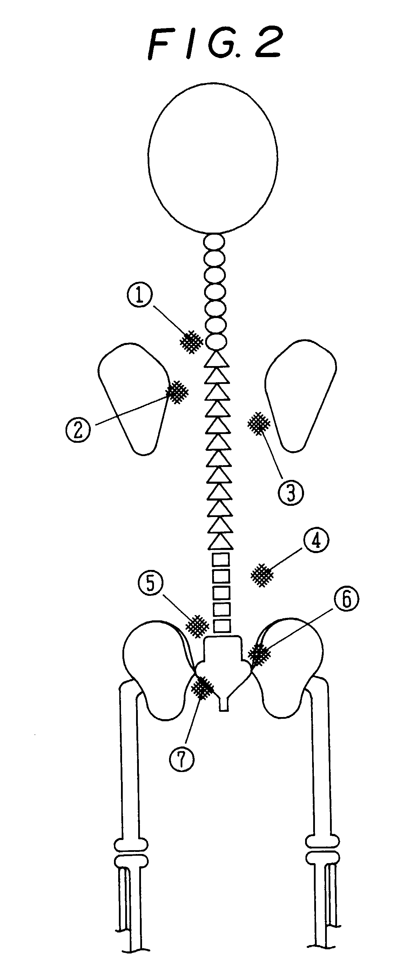

The comparison test was operated to two patients who complained of pain in their neck and waist.The healing degree of ailments in the neck and the waist can be verified by a measurement of a grasping power. The plasters were applied to the following seven points of the person's body as shown in Fig.2.

The first point ① indicates an application place remote from the seventh cervical vertebra to the left by two fingers width .

The second point ② indicates an application place remote from the third thoracic vertebrae to the right by four and a half fingers width .

The third point ③ indicates an application place remote from the fifth thoracic vertebrae to the right by four and a half fingers width .

The fourth point ④ indicates an application place remote from the second lumbar vertebrae to the right by four and a half fingers width .

The fifth point ⑤ indicates an application place remote from the fifth lumbar vertebrae to the left by two fingers width .

The sixth point ⑥ indicates the upper right of the epigaster.

The seventh point ⑦ indicates the lower left outside of the sacrum.

One patient is a male and 49 years old and the other patient is a female and 39 years old. The test was operated at thirty-second intervals.

As shown in table 1, considering from the measured values of the male patient,when he was applying the non-pored plasters to his body,the measured value of the grasping power of his left arm was 48.0 kg. In comparison with that value,when he applied the mesh-like plasters instead of the non-pored plasters to the designated same places on his body,the measured value of the grasping power of his left arm has improved to 49.5 kg.∼ 52.5 kg.

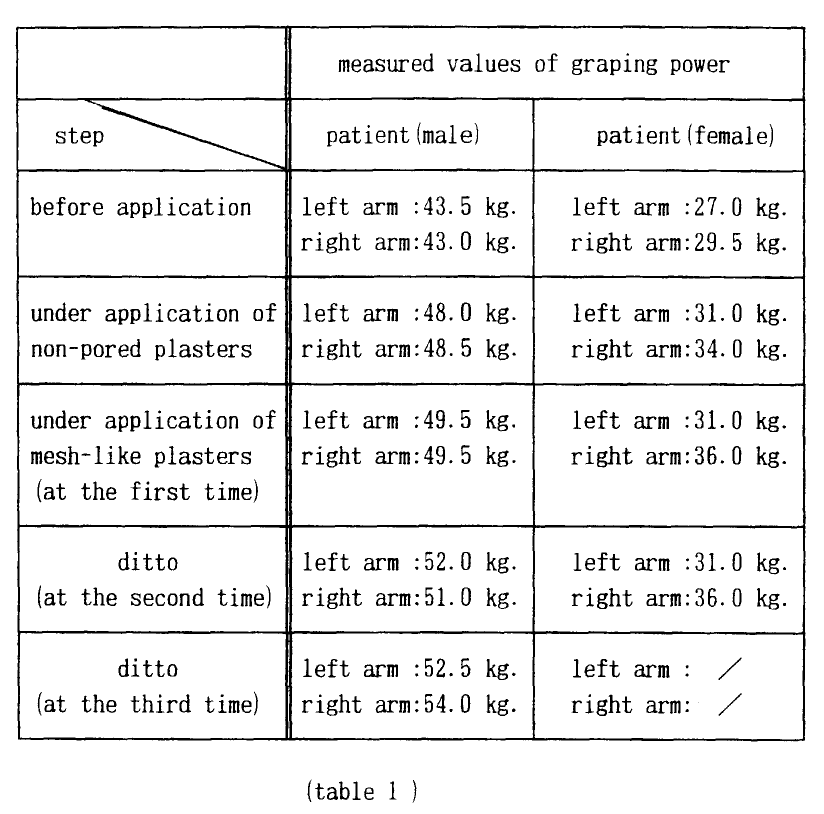

That is to say, even if the burden for his neck and waist became heavy,his muscles in said portions were able to withstand the heavy burden. This means that the trouble in his neck and waist has recovered. And in the case of his right arm, too, the measured value of the grasping power under application the former plaster is 48.5 kg.,and after application the plaster in this invention, the values were raised up to 49.5 kg.∼ 54.0 kg.

Besides, As for the female patient, too, it is obvious that the same effectiveness by the plaster in this invention as the male patient can be obtained

The effect of the invention is as follows;

As comparison with the non-pored plaster,the mesh-like plaster in this invention can cure a trouble in a muscles of the human body speedily and efficaciously .

The invention will be more fully understood from the following description given by way of example only with reference to the several figures of the accompanying drawings in which,

Fig.1 is a plan view of a plaster as an embodiment of this invention.

Fig.2 is a schematic illustration of the human body designating application places of the plaster in acomparison test.

Fig.3 is a schematical view of a process manufacturing a plaster.

Fig.4 is a sectional view of a plaster of Fig.1.

Fig.5 is a schematical view of a mesh-like plaster separated from a separate paper.

Fig.6 is plan views of the other plasters.

### [ EMBODIMENTS ]

Preferred embodiments of this invention are explained by the figures.

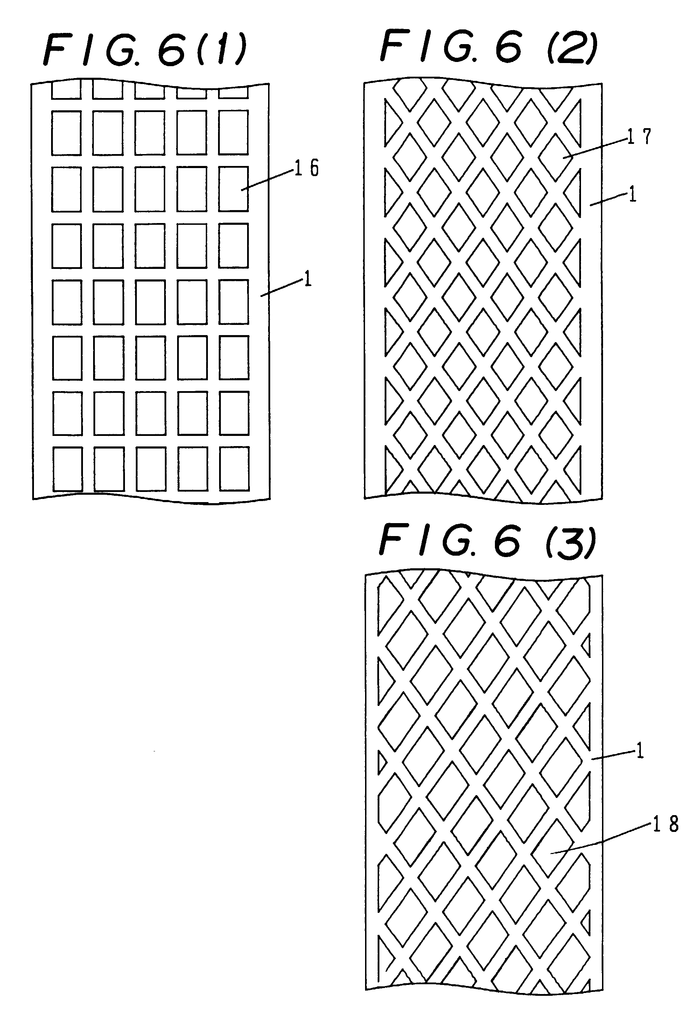

As shown in Fig.1 and Fig.4, a plaster in this invention is made of a mesh-like adhesive main sheet 1. In this embodiment, the main sheet 1 is 57 millimeters in width and perforated with many uniform square holes 10 arranged side by side. In other words,the main sheet 1 is a lattice-type sheet. The one side of the square hole 10 is 7 millimeters long.And the square holes 10 are arranged at 3-millimeter intervals. An adhesive face of the main sheet 1 is coverd by a separate paper 2.

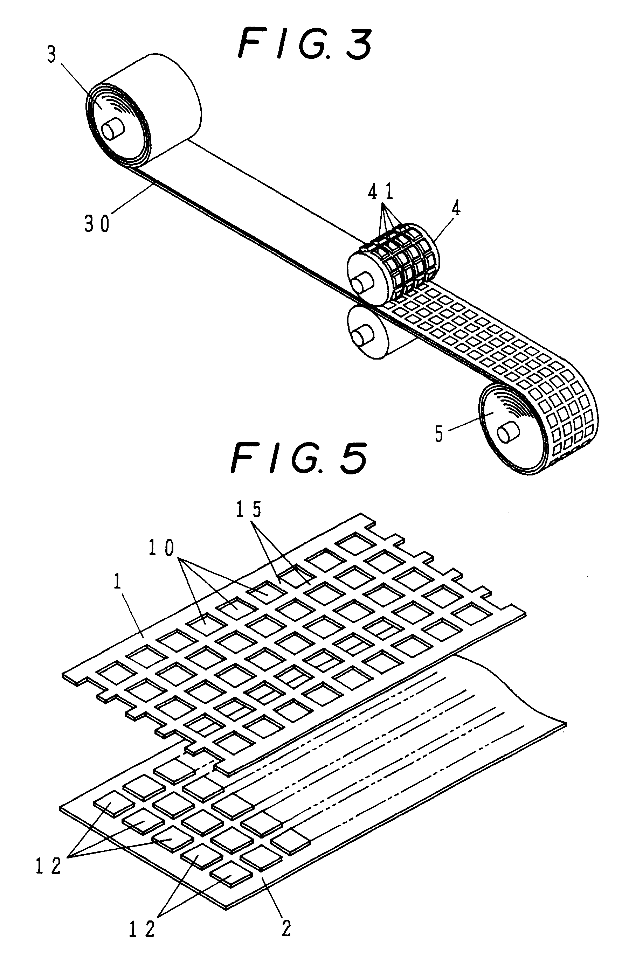

Next , a process of manifacturing of the mesh-like plaster will be explained with Fig.3 refering.

An original sheet 30 consists of the main sheet 1 having the adhesive face and the separate paper 2 covering the adhesive face of the sheet 1. The original sheet 30 is rolled up. And the roll of the original sheet 30 is called an original roll 3 .

The original sheet 30 is pulled out of the original roll 3 so that the main sheet 1 is on the separate paper 2. The punching roll 4 having many square cutting blades 41 is provided at a downstream of the original roll 3 which the original sheet 30 is pulled out .By means of the cutting blades 41,many square slittings which correspond to outlines of the square holes 10 are cut into only the main sheet 1 which is the upside of the original sheet 30. And after the punching,the original sheet 30 is rolled up again.Thus,a finished roll 5 is perfected.

When you need this plaster,you may pull the main sheet 1 having the square slittings and the separate paper 2 out of the finished roll 5 and may cut the sheet 1 in the necessary length. And when the separate paper 2 is separated from the adhesive face of the sheet 1 ,as shown in Fig.5, square scraps 12 which correspond to the square holes 10 are left over on the separate paper 2. Thus, the main sheet 1 is perforated with the square holes 10.

The length of the one side of the square hole 10 is not restricted to 7 millimeters. It is allowed in the range of from 3 millimeters to 7 millimeters. If the sizes of the hole 10 are within the above mentioned ranges,the remedial values by these plasters can be gained on the same level.

And the interval 15 between two square holes measures in the range of from 2.5 millimeters to 3 millimeters in width. And then,the widths of the interval 15 between two holes and the lengths of the one side of the hole 10 may be combined suitably within the above mentioned extent.

Besides,the shape of the hole perforated uniformly in the main sheet 1 is not restricted to square,any shape will do, for example, rectangle holes 16 as shown in Fig.6-(1), lozenge holes 17 as shown in Fig.6-(2), and parallelogram holes 18 as shown in Fig.6-(3), provided that the one side of the each hole measures in the range of from 3 to 7 millimeters, and that the interval between facing sides of the two holes measures in the ranges of from 2.5 to 3 millimeters.

## Claims

1. A method of manufacturing plasters for application to a troubled or damaged part of the human body in order to abate a muscular ache or swelling;
said plasters being made of a mesh-like sheet having an adhesive face and being perforated with a plurality of generally uniform holes (10, 16, 17, 18);
said method comprising the step of,
cutting a plurality of slits in sheet (30); and being characterised by the further steps of,
supplying said sheet which comprises main sheet (1) having an adhesive face and separate paper (2) covering the adhesive face; and
using a punching roll (4) in said cutting step to cut said plurality of slits in said main sheet (1) without cutting said separate paper (2), said plurality of slits corresponding to outlines of said generally uniform holes.

2. Amethod as claimed in claim 1 characterised in that, the step of supplying said sheet comprises the step of pulling said sheet (30) from a first roll (3); and by, the further step of, after said cutting step, rolling said sheet (30) to form a second roll (5).

3. A method as claimed in claim 1 or claim 2, characterised in that said plasters are perforated with uniform square holes (10).

4. A method as claimed in claim 1 or claim 2, characterised in that said plasters are perforated with uniform rectangular holes (16).

5. A method as claimed in claim 1 or claim 2, characterised in that said plasters are perforated with uniform lozenge-type holes (17).

6. A method as claimed in any one of claims 3 to 5, characterised in that one side of each hole measures in the range of from three to seven millimetres, and an interval between facing sides of the two adjacent holes measures in the range of from two point five to three millimetres.

7. A method as claimed in claim 3 or claim 5, characterised in that one side of each hole is seven millimetres long and facing sides of the two holes are arranged at three-millimetre intervals.
